# FASCICULE DE BREVET EUROPEEN

(11) **EP 4 088 659 B1**
(45) Date de publication et mention de la délivrance du brevet: **26.08.2026**
(21) Numéro de dépôt: 22172406.5
(22) Date de dépôt: 09.05.2022
(51) Int. Cl.: A61B 5/372, G06F 3/01

(54) **INTERFACE NEURONALE DIRECTE UTILISANT UN MODÈLE SEMI-MARKOVIEN HIÉRARCHISÉ**
DIREKTE NEURONALE SCHNITTSTELLE, DIE EIN HIERARCHISIERTES SEMI-MARKOW-MODELL VERWENDET
DIRECT NEURAL INTERFACE USING A SEMI-MARKOVIAN HIERARCHISED MODEL

(30) Priorité: 10.05.2021 FR 2104905
(43) Date de publication de la demande: 16.11.2022
(73) Titulaire: Commissariat à l'Energie Atomique et aux Energies Alternatives, 75015 Paris (FR)
(72) Inventeur: MARTEL, Felix, 38054 GRENOBLE CEDEX 09 (FR); AKSENOVA, Tetiana, 38054 GRENOBLE CEDEX 09 (FR); MOLY, Alexandre, 38054 GRENOBLE CEDEX 09 (FR)
(74) Mandataire: INNOV-GROUP

(56) Documents cités:
- EP-A1- 3 789 852
- CHUN ZHU ET AL: "Human intention recognition in Smart Assisted Living Systems using a Hierarchical Hidden Markov Model", AUTOMATION SCIENCE AND ENGINEERING, 2008. CASE 2008. IEEE INTERNATIONAL CONFERENCE ON, IEEE, PISCATAWAY, NJ, USA, 23 August 2008 (2008-08-23), pages 253 - 258, XP031321720, ISBN: 978-1-4244-2022-3
- AMIRIBESHELI MOHSEN ET AL: "A review of smart homes in healthcare", JOURNAL OF AMBIENT INTELLIGENCE AND HUMANIZED COMPUTING, SPRINGER BERLIN HEIDELBERG, BERLIN/HEIDELBERG, vol. 6, no. 4, 14 March 2015 (2015-03-14), pages 495 - 517, XP035522418, ISSN: 1868-5137, [retrieved on 20150314], DOI: 10.1007/S12652-015-0270-2

## Description

### DOMAINE TECHNIQUE

La présente invention concerne le domaine des interfaces neuronales directes encore dénommées BCI *(Brain Computer Interface)* ou BMI *(Brain Machine Interface).* Elle trouve notamment à s'appliquer à la commande neuronale directe d'une machine, telle qu'un exosquelette ou un ordinateur.

### ETAT DE LA TECHNIQUE ANTERIEURE

Les interfaces neuronales directes utilisent les signaux électro-physiologiques émis par le cortex cérébral pour élaborer un signal de commande. Ces interfaces neuronales ont fait l'objet de nombreuses recherches notamment dans le but de restaurer une fonction motrice à un sujet paraplégique ou tétraplégique à l'aide d'une prothèse ou d'une orthèse motorisée.

Les interfaces neuronales peuvent être de nature invasive ou non invasive. Les interfaces neuronales invasives utilisent des électrodes intracorticales (c'est-à-dire implantées dans le cortex) ou des électrodes corticales (disposées à la surface du cortex) recueillant dans ce dernier cas des signaux électrocorticographiques (ECoG). Les interfaces neuronales non invasives utilisent des électrodes placées sur le cuir chevelu pour recueillir des signaux électroencéphalographiques (EEG). D'autres types de capteurs ont été également envisagés comme des capteurs magnétiques mesurant les champs magnétiques induits par l'activité électrique des neurones du cerveau. On parle alors de signaux magnétoencéphalographiques (MEG).

Les interfaces neuronales directes utilisent avantageusement des signaux de type ECoG, présentant l'avantage d'un bon compromis entre biocompatibilité (matrice d'électrodes implantées à la surface du cortex) et qualité des signaux recueillis.

Les signaux ECoG ainsi mesurés doivent être traités afin d'estimer la trajectoire du mouvement désiré par le sujet et en déduire les signaux de commande de l'ordinateur ou de la machine. Par exemple, lorsqu'il s'agit de commander un exosquelette, l'interface BCI estime la trajectoire du mouvement désiré à partir des signaux électro-physiologiques mesurés et en déduit les signaux de contrôle permettant à l'exosquelette de reproduire la trajectoire en question. De manière similaire, lorsqu'il s'agit de commander un ordinateur, l'interface BCI estime par exemple la trajectoire souhaitée d'un pointeur ou d'un curseur à partir des signaux électro-physiologiques et en déduit les signaux de commande du curseur/pointeur.

L'estimation de trajectoire, et plus précisément celle des paramètres cinématiques (position, vitesse, accélération), est encore dénommée décodage neuronal dans la littérature. Le décodage neuronal permet notamment de commander un mouvement (d'une prothèse ou d'un curseur) à partir de signaux ECoG.

Lorsque les signaux ECoG sont acquis en continu, l'une des principales difficultés du décodage réside dans le caractère asynchrone de la commande, autrement dit dans la discrimination des phases pendant lesquelles le sujet commande effectivement un mouvement (périodes actives) des phases où il n'en commande pas (périodes de repos).

Pour contourner cette difficulté, des interfaces neuronales directes, dites synchrones, ne donnent la possibilité de commander un mouvement que pendant des fenêtres temporelles bien déterminées (par exemple des intervalles de temps se succédant périodiquement), signalées au sujet par un indice extérieur. Le sujet ne peut alors commander le mouvement que pendant ces fenêtres temporelles, ce qui s'avère prohibitif dans la plupart des applications pratiques.

Plus récemment, des interfaces neuronales directes à décodage continu ont été proposées dans la littérature. L'article de M. Velliste et al. intitulé « Motor cortical correlates of arm resting in the context of a reaching task and implications for prosthetic control » publié dans The Journal of Neuroscience, 23 avril 2014, 34(17), pp. 6011-6022, décrit notamment une interface neuronale directe dans laquelle les périodes de repos (*idle state)* sont détectées par LDA (Linear Discriminant Analysis) à partir de la fréquence d'émission des potentiels d'action (*neuron firing rate).* Les paramètres cinématiques sont estimés pendant les périodes actives au moyen d'un modèle à transition d'états, les états étant prédits au moyen d'un filtre dit de Laplace-Gauss. Toutefois, les résultats ont été obtenus pour des matrices de micro-électrodes et n'ont pu être reproduits pour des électrodes corticales classiques. En outre, les commutations entre les périodes de repos et les périodes actives se traduisent par des discontinuités dans le décodage du mouvement et donc des à-coups le long de la trajectoire.

Une autre approche a été proposée dans l'article de L. Srinivasam et al. intitulé « General-purpose filter design for neural prosthetic devices » publié dans J. Neurophysiol. Vol 98, pp. 2456-2475, 2007. Cet article décrit une interface neuronale directe à décodage continu dans laquelle la séquence des états actifs et des états de repos est générée par un modèle markovien du 1^{er} ordre. Un filtre de Kalman commuté ou SKF (Switching Kalman Filter) dont les matrices d'observation et les matrices de transitions dépendent de la variable de commutation cachée (état actif/ état de repos) permet d'estimer les paramètres cinématiques du mouvement. Ce type d'interface neuronale directe a été utilisé pour commander une chaise roulante à partir de signaux EEG sur la base de données de simulation mais n'a pas été testé pour des signaux ECoG. En outre, la détection d'état (état actif/ état de repos) est quelquefois erronée (taux élevé de faux positifs et faux négatifs).

Enfin, les interfaces neuronales directes précitées ont été développées pour décoder le mouvement d'un seul membre d'un sujet. Elles ne sont donc pas adaptées à la commande d'un exosquelette à plusieurs membres, notamment pour un sujet tétraplégique, paraplégique ou hémiplégique, ce qui limite sensiblement leur champ d'application.

Il a été proposé dans la demande publiée sous le numéro FR-A-3053495, de décoder le mouvement de plusieurs membres au moyen d'un mélange markovien d'experts ou MME *(Markov Mixture of Experts).* Ce décodage repose sur un modèle markovien caché ou HMM *(Hidden Markov Model),* un estimateur (ou expert) étant associé à chaque état caché du modèle.

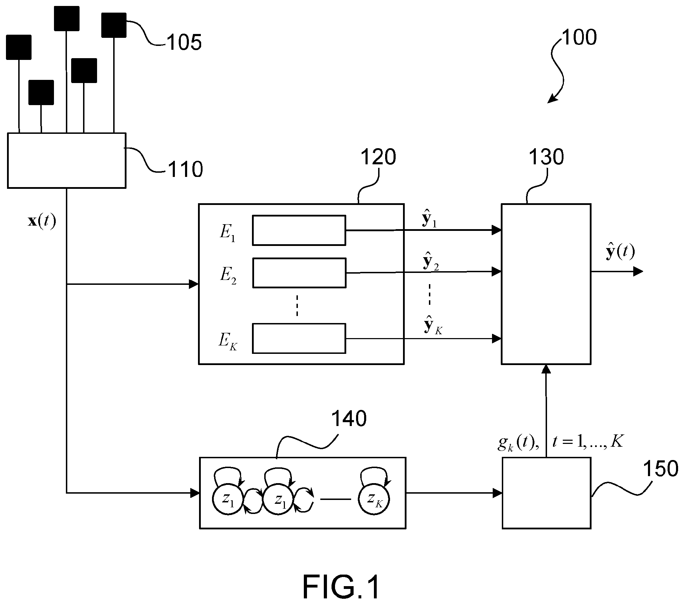

On a représenté schématiquement en Fig. 1, une interface BCI utilisant un décodage continu de mouvement par mélange markovien d'experts.

Cette interface, 100, fait intervenir, d'une part, un automate à états cachés, 140, pouvant prendre *K* états possibles et, d'autre part, une pluralité *K* d'estimateurs, 120, chaque estimateur (ou expert) étant associé à un état caché.

Les estimations des différents experts sont combinées dans un module de combinaison *(gating network),* 130, pour donner une estimation, ŷ(*t*) de la variable à expliquer, y(*t*), ici les paramètres cinématiques du mouvement, à partir de l'observation x(*t*) représentant les caractéristiques des signaux neuronaux à l'instant *t*, captés au moyen des électrodes 105. La séquence des observations x[1:*t*]={x(1), x(2),..., x(*t*)} est modélisée par un processus markovien sous-jacent, de premier ordre, à émission continue.

Si la variable d'entrée x(*t*) est de dimension *M* et la réponse y(*t*) de dimension *N*, et si les modèles prédictifs des différents experts *Eₖ*, sont choisis linéaires, autrement dit *Eₖ*(x(*t*)) *=* β*ₖ*x(*t*) où β*ₖ* est une matrice de taille *N* ×*M,* l'estimation par mélange d'experts s'exprime comme suit :
[Math. 1] $\hat{y} \left(t\right) = {\sum }_{k = 1}^{K} {g}_{k} \left(t\right) {β}_{k} x \left(t\right)$ où *gk*(*t*) sont les coefficients de pondération (ou de mélange) vérifiant ${\sum }_{k = 1}^{K} {g}_{k} \left(t\right) = 1$.

Comme indiqué dans la demande précitée, les paramètres du modèle HMM, c'est-à-dire les probabilités de transition entre états cachés, et les matrices β*ₖ* peuvent être estimés lors d'une phase de calibration, au moyen d'une méthode d'estimation supervisée. Dans ce cas, l'automate HMM et les *K* experts sont entraînés indépendamment les uns des autres. En particulier, chaque expert *Eₖ* peut être entrainé sur une sous-séquence d'observations correspondant à l'état *k*, la matrice β*ₖ* du modèle prédictif étant alors estimée au moyen d'une régression PLS *(Partial Least Squares)* linéaire à partir de cette sous-séquence.

De manière plus générale, la relation linéaire (1) peut être étendue à une relation multilinéaire. La variable d'entrée peut être alors représentée sous forme tensorielle ${X}_{_} ∈ {ℝ}^{{I}_{1} \times … \times {I}_{n}}$ où *n* est l'ordre du tenseur d'entrée et *Iᵢ* est la dimension du mode *i*.

Les modes du tenseur d'entrée peuvent être par exemple le temps (nombre d'échantillons dans le temps), la fréquence (nombre de bandes spectrales d'analyse), l'espace (nombre d'électrodes). De même, la réponse peut être représentée sous forme tensorielle ${Y}_{_} ∈ {ℝ}^{{J}_{1} \times … \times {J}_{m}}$ où *m* est l'ordre du tenseur de sortie. Les modes du tenseur de sortie peuvent correspondre à différents effecteurs, agissant par exemple sur différentes articulations d'un exosquelette.

Les tenseurs des modèles prédictifs des différents experts peuvent alors être estimés par une méthode PLS multivariée ou NPLS *(N-way PLS)* comme décrit dans la demande publiée sous le numéro FR-A-3046471.

Quels que soient les modèles prédictifs (PLS ou NPLS) des différents experts, on a pu constater qu'en raison de l'absence de stationnarité des signaux dans le cerveau humain, la durée de validité de ces modèles était relativement limitée. Il est par conséquent nécessaire de les mettre à jour régulièrement en procédant à de nouvelles phases de calibration. Or, chaque nouvelle phase de calibration nécessite de traiter une quantité de données très importante, combinant les données des calibrations antérieures et celles de la nouvelle calibration, et ce pour les différents experts. La durée de la mise à jour est alors souvent telle qu'elle requiert une interruption du fonctionnement de l'interface neuronale directe (calibration dite *off-line).* Il a été ainsi proposé dans la demande FR-A-3061318 une méthode itérative de calibration d'une interface neuronale directe basée sur un modèle prédictif NPLS. Cette méthode itérative de calibration a également été décrite dans l'article de A. Eliseyev et al. intitulé « Recursive exponentially weighted N-way Partial Least Squares regression with recursive validation of hyper-parameters in Brain Computer Interface applications » publié dans Nature, Scientific Reports, volume 7, article number 16281, publié le 24.11.2017. Cependant, cette méthode de calibration itérative ne s'applique pas à une interface neuronale directe utilisant un mélange markovien d'experts tel que décrit dans la demande FR-A-3046471 précitée.

Il a été proposé dans la demande EP-A-3789852 une interface neuronale directe à décodage continu utilisant un mélange d'experts, le modèle prédictif de chaque expert étant associé un état d'observation et étant entrainé, dans une phase de calibration, sur une sous-séquence correspondant à cet état, selon une méthode de régression REW-NPLS. Un second modèle prédictif donnant la probabilité d'occupation de chaque état du modèle HMM est également entrainé au moyen d'une méthode de régression REW-NPLS et permet de calculer les coefficients de pondération du mélange markovien.

Une telle interface neuronale directe, désignée dans la littérature par l'acronyme REW-MSLM *(Recursive Exponentially Weighted Markov Switching Multi-Linear Model),* hérite, d'une part, des caractéristiques d'un décodage REW-NPLS et, d'autre part, de celles d'un mélange d'experts commandé par un modèle HMM comme décrit en relation avec la Fig. 1.

Une interface neuronale directe REW-MSLM est brièvement décrite ci-après. Celle-ci fournit un tenseur de commande à partir d'un tenseur d'observation.

Le tenseur représentant la variable d'entrée, dit tenseur d'entrée ou tenseur d'observation, est d'ordre *n*+1, le premier mode étant celui relatif aux instants d'observation *(epochs).* Le tenseur d'entrée (ou tenseur d'observation) est noté **X** et est de dimension *N*×*I*₁×...×*Iₙ*.

De la même façon, la trajectoire du mouvement imaginé, observé ou réalisé est décrite par un tenseur de sortie (ou tenseur de commande), d'ordre *m* + 1, noté **Y**, de dimension *N*×*J*₁×...×*Jₘ*, dont le premier mode correspond aux instants successifs auxquels s'appliqueront les commandes, les autres modes correspondant aux commandes de différents effecteurs ou aux différents degrés de liberté d'un robot multi-axe.

Le tenseur de sortie fournit *N* blocs consécutifs de données de commande, chacun des blocs permettant de générer les signaux de commande relatifs aux différents effecteurs ou degrés de liberté. Ainsi, l'homme du métier comprendra que la dimension de chaque bloc de données pourra dépendre du cas d'usage envisagé et notamment du nombre de degrés de liberté de l'effecteur.

On note dans la suite **X***ₜ* le tenseur d'observation à l'instant *t*. Ce tenseur est par conséquent d'ordre *n* et de dimension *I*₁×...×*Iₙ.* Il prend ses valeurs dans un espace ${X}_{_} ⊂ {ℝ}^{{I}_{1} \times … \times {I}_{n}}$ où 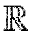 est l'ensemble de réels. De manière similaire, on note **Y***ₜ* le tenseur de commande à l'instant *t*. Ce tenseur de sortie est par conséquent d'ordre *m* et de dimension *J*₁×...×*Jₘ*. Il prend ses valeurs dans un espace ${Y}_{_} ⊂ {ℝ}^{{J}_{1} \times … \times {J}_{m}}$.

On suppose que l'espace *X* est formé par l'union d'une pluralité *K* de régions, non nécessairement disjointes. Autrement dit, ${X}_{_} = {∪}_{k = 1}^{K} {X}_{k}$ où *Xₖ, k* = 1,..., *K* sont les régions en question.

L'interface neuronale directe REW-MSLM utilise un mélange d'experts, chaque expert *Eₖ* opérant sur la région élémentaire *Xₖ* de l'espace des caractéristiques d'entrée et étant capable de prédire le tenseur de commande **Y***ₜ* à l'instant *t* à partir du tenseur d'observation, **X***ₜ*, lorsque ce dernier appartient à *Xₖ.* Autrement dit, à chaque région *Xₖ* correspond un modèle de prédiction du tenseur de commande **Y**_{*t*à} partir du tenseur d'observation **X***ₜ.* Un expert *Eₖ* peut être ainsi considéré comme une application multilinéaire de *Xₖ* dans *Y*.

On suppose par ailleurs que chaque expert *Eₖ* est associé à un état caché*k* d'un modèle Markovien du premier ordre (HMM). Les différents experts sont combinés à l'aide de coefficients de combinaison dépendant de l'état caché au moment de la prédiction. En définitive, à partir d'un tenseur d'entrée (ou d'observation) **X***ₜ*, l'interface neuronale estime le tenseur de sortie (ou de commande) **Y***ₜ*, au moyen de :
[Math. 2] ${\hat{{Y}_{_}}}_{t} ∈ {\sum }_{k = 1}^{K} {γ}_{k}^{t} \left({{β}_{_}}_{k}{{X}_{_}}_{t}+{{δ}_{_}}_{k}\right)$ où **β***ₖ*, **δ***ₖ* sont respectivement un tenseur de coefficients de prédiction et un tenseur de biais de prédiction de l'expert *Eₖ* et ${γ}_{k}^{t}$ est le coefficient de pondération (encore dénommé *gating coefficient)* relatif à cet expert et à l'instant *t* considéré. Le coefficient de pondération ${γ}_{k}^{t}$ n'est autre que la probabilité conditionnelle que le modèle HMM soit dans l'état *k* connaissant les tenseurs d'entrée passés **X**_{1:*t*} = **X**₁, **X**₂, ..., **X***ₜ*.

L'ensemble des coefficients et des biais de prédiction, dénommés collectivement paramètres de prédiction des différents experts est désigné par θ*ₑ* = {(β*ₖ*,δ*ₖ*)|*k* = 1,.., *K*}. L'ensemble de paramètres relatifs à la combinaison des différents experts (incluant les paramètres du modèle HMM sous-jacent) est désigné quant à lui par θ_{g} = {*A,*{*dₖ* |*k* = 1,..,*K*}*,π*} où A est la matrice de transition entre états, de taille *K* × *K*, c'est-à-dire la matrice dont les éléments *a^{ij}* (indépendants du temps par hypothèse du modèle HMM) sont définis par *a^{ij}* = *p*(*zₜ* = *j*|*z*_{*t*-1} = *i*) où *zₜ* représente l'état du modèle à l'instant *t* et *z*_{*t*-1} représente l'état du modèle à l'instant précédent ; {*dₖ* |*k =* 1,.., *K*} les paramètres permettant de déterminer la probabilité conditionnelle d'observer le tenseur d'entrée **X***ₜ* connaissant l'état *zₜ* = *k*, et *π* est un vecteur de taille *K* donnant les probabilités d'occupation des différents états à l'instant initial, autrement dit *πᵢ* = *p*(*zₜ* = *i*)_{*t*=0}

Le décodeur REW-MSLM est entrainé pendant des phases de calibration indicées par l'indice *u*, chaque phase de calibration faisant intervenir une pluralité Δ*L* d'instants successifs issus de la séquence d'observation. On note **X***ᵤ* le tenseur de dimension Δ*L*×*I*₁ × ...× *Iₙ* regroupant les tenseurs d'entrée pendant la phase de calibration *u* , et **Z***ᵤ* ∈ {0,1}^{*K*×Δ*L*} la matrice binaire donnant, à chaque instant, l'état latent du modèle HMM (la matrice possédant un seul élément égal à 1 par colonne indiquant l'état actif parmi les *K* états possibles).

L'interface neuronale directe REW-MSLM permet de contrôler plusieurs effecteurs agissant sur différentes articulations d'un exosquelette, un état pouvant être associé à une articulation par exemple. En outre, elle permet de garantir la stabilité d'un état au repos (*idle state)* prolongé et un contrôle asynchrone des effecteurs.

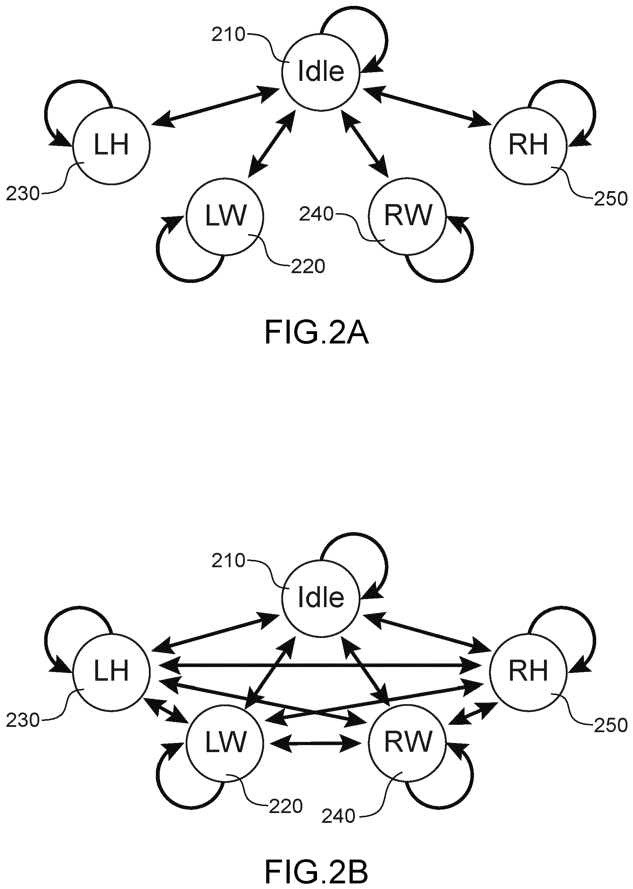

La Fig. 2A représente un premier exemple de diagramme d'états pouvant être utilisé par l'interface neuronale directe de la Fig. 1.

Ce diagramme d'états modélise les mouvements d'un exosquelette, à savoir les mouvements d'un poignet gauche (LW), d'une main gauche (LH), d'un poignet droit (RW) et d'une main droite (RH).

On a représenté en 210 un état de repos de l'exosquelette, en 220 un état actif poignet gauche, en 230 un état actif de la main gauche, en 240 un état actif du poignet droit et en 250 un état actif de la main droite. Les transitions entre états ont été représentées par des flèches.

On note que dans un tel diagramme d'états, pour passer d'un mouvement du poignet gauche à celui de la main gauche par exemple, il faut passer par l'état de repos. Ce diagramme d'états est très simple et favorise excessivement l'état de repos au détriment des états actifs. S'il présente l'avantage de réduire le taux de mauvaise détection des états actifs les plus rares lorsque le nombre d'états est élevé, il ne permet pas d'effectuer des mouvements fluides.

En effet, le passage obligé par l'état de repos conduit à des temps de latence importants entre états actifs, ce qui pénalise la réactivité de la réponse aux signaux électrophysiologiques.

Une solution possible pour accroître la réactivité de cette réponse est d'utiliser un diagramme d'états tel que celui représenté en Fig. 2B.

Les éléments portant les mêmes numéros de référence sont identiques à ceux de la Fig. 2A.

Ce diagramme d'états est basé sur un graphe complet, autrement dit dans lequel tous les états sont connectés 2 à 2. Ce graphe peut devenir particulièrement complexe lorsque le nombre d'états est élevé, par exemple lorsque les articulations sont nombreuses. Dans ce cas, l'apprentissage est lent étant donné que chaque transition doit être entrainée. En outre, le second modèle prédictif, c'est-à-dire le classificateur d'états, peut commettre des erreurs de détection conduisant à un dysfonctionnement de l'exosquelette. Enfin, lorsque l'exosquelette doit effectuer des tâches complexes mettant en jeu de nombreux états, le temps de latence pour détecter un nouvel état sans erreur peut être relativement long, ce qui est préjudiciable à sa vitesse de réponse et peut interdire des mouvements rapides.

Un but de la présente invention est par conséquent de proposer une interface neuronale directe de type REW-MSLM qui ne présente pas les inconvénients précités, à savoir qui autorise des mouvements complexes mettant en œuvre une pluralité d'effecteurs, tout en assurant un faible temps de réponse du système.

### EXPOSE DE L'INVENTION

La présente invention est définie par une d'une interface neuronale directe destinée à recevoir une pluralité de signaux électrophysiologiques émis par le cortex cérébral d'un usager acquis au moyen d'une pluralité de capteurs et à fournir des signaux de commande décrivant une trajectoire à réaliser, lesdits signaux électrophysiologiques subissant un prétraitement pour obtenir un tenseur d'observation (**X***ₜ*) en chaque instant d'observation, dans laquelle l'évolution du tenseur d'observation est modélisée par un modèle HMM hiérarchisé, dit H2M2, comprenant une pluralité de sous-modèles HMM organisés selon une arborescence hiérarchique, ladite arborescence comportant à sa racine un modèle principal comprenant un état de repos , chaque état d'un sous-modèle HMM étant soit un état interne auquel est associé un sous-modèle HMM de plus bas niveau dans l'arborescence, soit un état de production, ladite interface utilisant un mélange d'experts, chaque expert (*Eₛ*) étant associé à un état de production et étant défini par un modèle prédictif multilinéaire, les prédictions de chaque expert étant combinées au moyen de coefficients de mélange (*γ_{s,t}*, γ_{*SMs,t*;} *s* ∈ *S_{prod}*) pour fournir un tenseur de commande (**Ŷ***ₜ*) représentant lesdits signaux de commande.

Ladite arborescence comporte par exemple un état latéral droit et un état latéral gauche correspondant respectivement à la partie droite et à la partie gauche du corps de l'usager, une première branche de l'arborescence étant associée à l'état latéral droit et une seconde branche de l'arborescence étant associée à l'état latéral gauche

Avantageusement, le tenseur de commande **Y***ₜ* test estimé à partir du tenseur d'observation **X***ₜ* au moyen de ${\hat{{Y}_{_}}}_{t} = {\sum }_{s ∈ {S}_{prod}} {γ}_{s , t} \left({{B}_{_}}_{s}{{X}_{_}}_{t}+{{b}_{_}}_{s}\right)$ où **B**ₛₑₜ **b**ₛ sont respectivement les tenseurs des coefficients de prédiction et des biais de prédiction du modèle prédictif multilinéaire définissant l'expert associé à l'état de production Set *γ_{s,t}* est un coefficient de mélange caractéristique de la probabilité d'occupation de l'état de production *s* à l'instant *t*.

Dans une phase de calibration, chacun des experts, *Eₛ*, est entrainé selon une méthode de régression REW-NPLS, sur une sous-séquence du tenseur d'observation et du tenseur de commande de consigne, correspondant à l'état *s* ∈ *S_{prod}*.

Dans ladite phase de calibration, les coefficients de mélange des différents experts peuvent être estimés au moyen d'une méthode de régression REW-NPLS.

Avantageusement, au moins certains des sous-modèles HMM de l'arborescence sont semi-markoviens.

Dans ce cas, le tenseur de commande **Y***ₜ* peut être estimé à partir du tenseur d'observation **X***ₜ* au moyen de ${\hat{{Y}_{_}}}_{t} = {\sum }_{s ∈ {S}_{prod}} {γ}_{{SM}_{s , t}} \left({{B}_{_}}_{s}{{X}_{_}}_{t}+{{b}_{_}}_{s}\right)$ où **B**ₛₑₜ **b**ₛ sont respectivement les tenseurs des coefficients de prédiction et des biais de prédiction du modèle prédictif multilinéaire définissant l'expert associé à l'état de production *s* et *γ_{SMs,t}* est un coefficient de mélange caractéristique de la probabilité d'occupation de l'état de production *s* à l'instant *t*, la probabilité qu'un état de production du modèle soit actif à l'instant *t* étant donnée par ${γ}_{t} = \left({A}_{SM}\left(t\right){γ}_{t - 1}\right) ∘ {γ}_{t}^{emission}$ où *γ_{s,t}* est un vecteur dont les éléments sont les probabilités *γ_{s,t}* respectives que les états de production soient actifs, ${γ}_{t}^{emission}$ est un vecteur donnant les probabilités d'émission des différents états de production et ∘ est le produit de Hadamard.

La probabilité de transition entre un état de départ et un état d'arrivée d'un sous-modèle HMM semi-markovien est choisie d'autant plus élevée que la durée d'occupation de l'état de départ est plus grande.

Avantageusement, les probabilités de transition entre états d'un sous-modèle HMM semi-markovien évoluent dans le temps selon une loi paramétrée par une pluralité de paramètres d'évolution temporelle, lesdits paramètres d'évolution temporelle étant estimés dans une phase de calibration supervisée pendant laquelle l'usager reçoit des instructions de cadence prédéterminée pour effectuer un ou des mouvement(s) de manière répétitive, la durée d'occupation de chaque état du sous-modèle HMM semi-markovien étant enregistrée au cours du temps.

Alternativement, les probabilités de transition entre états d'un sous-modèle HMM semi-markovien évoluent dans le temps selon une loi paramétrée par une pluralité de paramètres d'évolution temporelle, lesdits paramètres d'évolution temporelle étant estimés, dans une phase de calibration semi-supervisée et/ou une phase opérationnelle, à partir du tenseur d'observation, la durée d'occupation de chaque état du sous-modèle HMM semi-markovien étant enregistrée au cours du temps.

Dans un exemple de réalisation, les signaux électrophysiologiques sont des signaux ECoG et les signaux de commande sont destinés à commander les actuateurs d'un exosquelette.

### BREVE DESCRIPTION DES DESSINS

D'autres caractéristiques et avantages de l'invention apparaîtront à la lecture d'un mode de réalisation préférentiel de l'invention, décrit en référence aux figures jointes parmi lesquelles :
[Fig. 1], déjà décrite, représente de manière schématique une interface neuronale directe utilisant un mélange markovien d'experts, connue de l'état de la technique ;
[Fig. 2A], déjà décrite, représente un premier exemple de diagramme d'états pouvant être utilisé par l'interface neuronale directe de la Fig. 1 ;
[Fig. 2B], déjà décrite, représente un second exemple de diagramme d'états pouvant être utilisé par l'interface neuronale directe de la Fig. 1 ;
[Fig. 3A] représente l'architecture générale d'un modèle hiérarchique markovien à états cachés (H2M2) pouvant être utilisé par une interface neuronale directe selon un premier mode de réalisation de l'invention ;
[Fig. 3B] représente de manière schématique un exemple détaillé de diagramme d'états d'un modèle un modèle hiérarchique markovien à états cachés (H2M2) pouvant être utilisé par une interface neuronale directe selon un premier mode de réalisation de l'invention ;
[Fig. 4] représente de manière schématique un interface neuronale directe utilisant un mélange d'experts contrôlé par un modèle hiérarchique (semi) markovien à états cachés selon un premier (resp. un second) mode de réalisation de l'invention.

### EXPOSE DETAILLE DE MODES DE REALISATION PARTICULIERS

On considérera dans la suite une interface neuronale directe de type REW-MSLM telle que décrit dans la partie introductive. On rappelle que cette interface utilise un automate à états cachés, basé sur modèle HMM, pouvant prendre *K* états possibles, ainsi qu'une pluralité d'experts *Eₖ, k* = 1, ..., *K*, utilisant un premier modèle prédictif, chaque expert étant associé à un état caché. Chaque expert *Eₖ* est entrainé, dans une phase de calibration, selon une méthode de régression REW-NPLS, sur une sous-séquence du tenseur d'observation et du tenseur de commande de consigne, correspondant à l'état *k.* Le classificateur d'état, donnant la probabilité d'occupation de chaque état du modèle HMM, utilise un second modèle prédictif et est également entrainé, pendant une phase de calibration, au moyen d'une méthode de régression REW-NPLS.

Une première idée à la base de la présente invention est d'utiliser un automate à états cachés basé sur un modèle markovien hiérarchisé, dénommé HHMM *(Hierarchical Hidden Markov Model)* ou plus simplement H2M2, organisé selon une structure arborescente à partir d'une racine commune correspondant à l'état de repos (*idle state).* L'arbre des états cachés pourra notamment comprendre une première branche correspondant à la partie droite du corps de l'usager et une seconde branche correspondant à la partie gauche de son corps, les deux branches étant issues de la racine commune. D'autres structures arborescentes pourront être envisagées par l'homme du métier sans sortir du cadre de la présente invention. Par exemple, l'arbre des états cachés pourra comporter une première branche correspondant à la main droite (se divisant elle-même en sous-états correspondant à sa position, sa rotation, son caractère ouvert/fermé), une seconde branche (avec la même division), et une troisième branche se divisant elle-même en un premier sous-état correspondant à la jambe droite et un sous état correspondant à la jambe gauche.

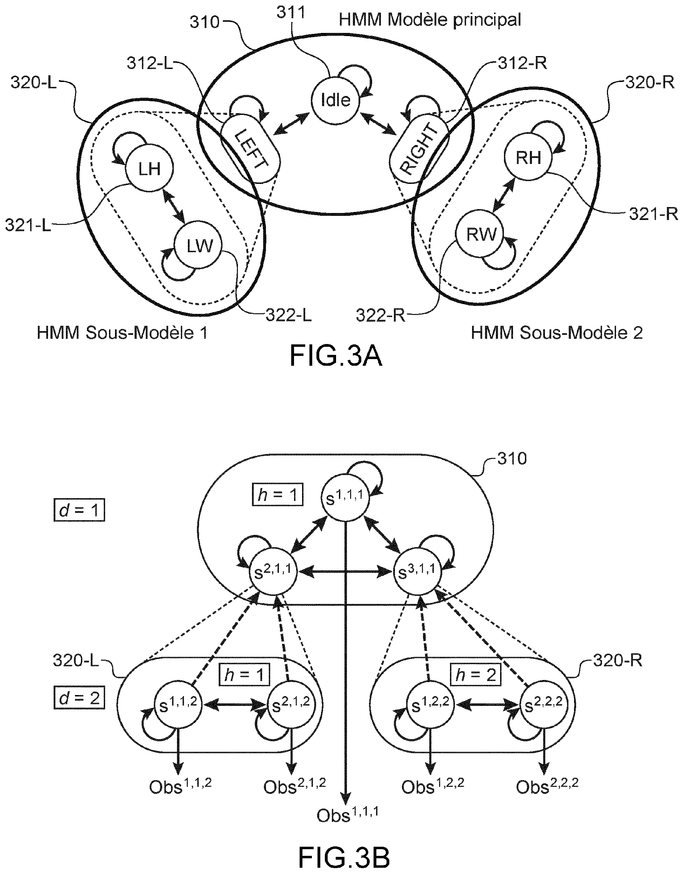

La Fig. 3A représente de manière schématique un exemple d'architecture d'un modèle hiérarchique markovien à états cachés (H2M2) pouvant être utilisé par une interface neuronale directe selon un premier mode de réalisation de l'invention.

Ce modèle comprend un modèle HMM principal, 310, dit modèle de rang 1, et deux sous-modèles HMM, dits encore modèles de rang 2, un sous-modèle 320-R, étant associé à la partie droite du corps et un sous-modèle, 320-L, étant associé à la partie gauche. Le modèle principal comprend un état de repos, 311, un premier état interne correspondant à un mouvement de la partie droite du corps, 312-R, et un second état interne, 312-L, correspondant à un mouvement de la partie gauche du corps de l'usager. A chaque état interne est associé un sous-modèle HMM latéral : ainsi, le sous-modèle 320-R est associé à l'état 312-R et le sous-modèle 320-L est associé à l'état 312-L du modèle principal. Le sous-modèle 320-R comprend ici un premier état 321-R associé au poignet droit et un second état 322-R associé à la main droite. De même, le sous-modèle 320-L comprend ici un premier état, 321-L, associé au poignet gauche, et un second état 322-L associé à la main gauche. Lorsqu'un état du sous-modèle 320-R est actif, cela suppose que l'état interne, 312-R, du modèle principal l'est aussi. De manière similaire, lorsqu'un état du sous-modèle 320-L est actif, cela suppose que l'état interne, 312-L du modèle principal l'est aussi.

De manière générale, chaque état du modèle H2M2 peut être soit un état interne, soit un état de production. Chaque état interne d'un modèle de rang *d* est associé à un sous-modèle HMM de rang *d* + 1, et chaque état de production génère une observation avec une probabilité d'émission donnée.

Ainsi, par exemple, l'état 322-R associé à la main droite peut être un état de production générant une observation soit un état interne associé à un sous-modèle de rang *d* = 3 comportant un état pour chaque doigt. Un état associé à un doigt peut être lui-même être associé à un sous-modèle de rang *d* = 4, comportant un état pour chaque phalange.

On comprend ainsi que le modèle H2M2 présente une structure arborescente pouvant décrire, par exemple, la dépendance hiérarchique des articulations d'un exosquelette.

La Fig. 3B représente un exemple de diagramme d'états d'un modèle H2M2 pouvant être utilisé dans le cadre de la présente invention.

Dans le cas présent, il s'agit d'un modèle H2M2 à deux niveaux hiérarchiques. Le modèle HMM de rang 1 (*h* = 1) comprend trois états notés respectivement *s*^{1,1,1}, *s*^{2,1,1}, *s*^{3,1,1}. L'état *s*^{1,1,1} correspond à l'état de repos, l'état *s*^{2,1,1} est un état interne correspondant à la partie gauche du corps et l'état *s*^{3,1,1} est un état interne correspondant à sa partie droite.

A l'état *s*^{2,1,1} est associé un sous-modèle HMM de rang 2 comprenant deux états de production, *s*^{1,1,2}. De même à l'état *s*^{3,1,1} est associé un sous-modèle HMM de rang 2, comprenant deux états de productions *s*^{1,2,2}, *s*^{2,2,2}.

De manière générale, les états du modèle H2M2 pourront être notés sous la forme indexée *s^{e,h,d}* où *d* est un indice donnant le rang (ou le niveau) de l'état dans l'arborescence (ou, de manière équivalente, le rang du sous-modèle HMM auquel il appartient dans l'arborescence), *h* est l'indice du sous-modèle dans le niveau en question, et *e* est un indice indexant l'état dans le sous-modèle. Les sous-modèles sont quant à eux notés sous la forme *HMM^{h,d}* où *d* indique le niveau hiérarchique auquel le modèle se situe dans l'arborescence, et *h* est l'indice du sous-modèle dans le niveau. L'arborescence est ainsi organisée en niveaux ou couches, indexé(e)s par l'indice *d*.

Chaque sous-modèle *HMM^{h,d}* est caractérisé par sa matrice de transition entre états ${A}^{h , d} = \left({a}_{i , j}^{h , d}\right) ∈ {ℝ}^{{K}_{h , d} \times {K}_{h , d}}$ où *K_{h,d}* est le nombre d'états dans le sous-modèle *HMM^{h,d}* et ${a}_{i , j}^{h , d}$ est la probabilité de transition ${a}_{i , j}^{h , d} = p \left({s}^{i , h , d}\left|{s}^{j , h , d}\right.\right)$ de l'état *s^{j,h,d}* à l'état *s^{i,h,d}*.

La probabilité initiale d'occupation de chaque état *s^{e,h,d}* du sous-modèle *HMM^{h,d}* est notée *π^{e,h,d}*.

Enfin, l'ensemble des états de production du modèle H2M2 est noté *S_{prod}* et celui des états internes est noté *Sᵢₙₜ* où *S* = *S_{prod}* ∪ *Sᵢₙₜ* est l'ensemble des états du modèle. Si l'on note *zₜ* a variable indiquant l'état de production à l'instant *t*, **X***ₜ* le tenseur d'observation en cet instant, la probabilité d'émission de cet état n'est autre que *p*(**X***ₜ* |*zₜ*).

On définit en outre, pour tout état actif, *s^{e,h,d}* de la couche *d* > 1, *ψ*(*s^{e,h,d}*) = *s*^{*e',d;,h'*-1} l'état actif appartenant à la couche précédente. Ainsi, pour un état de production *zₜ* à l'instant *t*, appartenant à la couche *d*, l'ensemble *Zₜ* = {*zₜ,ψ*(*zₜ*),*ψ*²(*zₜ*),..., *ψ^{d}*(*zₜ*)} définit un chemin d'états actifs dans l'arborescence du modèle H2M2 conduisant à l'état de production.

La probabilité d'activation d'un état *Sde* Sà l'instant *t* est alors définie par :
[Math. 3] ${γ}_{s , t} = p \left({z}_{t}=s\left|{{X}_{_}}_{1 : t}\right.\right) = {\prod }_{j = 0}^{card \left({Z}_{t}\right)} p \left({ψ}^{j}\left({z}_{t}\right)\left|{{X}_{_}}_{1 : t}\right.\right)$ où *card*(*Zₜ*) est le cardinal de l'ensemble *Zₜ* et où l'on a pris pour convention *ψ*⁰(*zₜ*)=*zₜ.*

Les coefficients de pondération *(gating coefficients), γ_{s,t}* des estimations fournies par les différents experts sont calculés au moyen d'une méthode de régression REW-NPLS adaptée au décodage discret comme décrit plus loin.

Plus précisément, à chaque phase de calibration *u*, ces coefficients de pondération sont mis à jour à partir de l'ensemble de données d'apprentissage (**X***ᵤ*,**Z***ᵤ*), où **X***ᵤ* est le tenseur d'entrée de dimension Δ*L*×*I*₁×...× *Iₙ* et Z*ᵤ* ∈ {0,1}^{*K*×Δ*L*} est la matrice binaire, tels que définis dans la partie introductive. On rappelle que est le nombre d'instants successifs intervenant pris en compte dans la phase de calibration.

Les éléments de chacune des matrices de transition A*^{h,d}* des modèles *HMM^{h,d}* sont mis à jour de la manière suivante :
[Math. 4] ${a}_{i , j}^{h , d} \left(u\right) = {λ}^{h , d} {a}_{i , j}^{h , d} \left(u-1\right) + \frac{{ν}_{i , j}^{h , d} \left(u\right)}{{E}^{h , d}}$ où ${a}_{i , j}^{h , d} \left(u\right)$, resp. ( ${d}_{\hat{i} , j}^{h , d} \left(u-1\right)$), l'élément en ligne *i* et colonne *j* de la matrice de transition A*^{h,d}* au terme de la phase de calibration *u*, 0 < *λ^{h,d}* < 1 est un coefficient d'oubli relatif au sous-modèle *HMM^{h,d}.* Le cas échéant, ces coefficients d'oubli peuvent être choisis identiques ou bien décroissants avec le niveau hiérarchique *d* (ainsi un modèle profond, c'est-à-dire près de la racine, est mis à jour plus lentement qu'un modèle moins profond). *E^{h,d}* est le nombre d'états du sous-modèle *HMM^{h'd}* et ${ν}_{i , j}^{h , d} \left(u\right)$ est le nombre de transitions de l'état *i* vers l'état *j* pendant la phase de calibration *u*. Il convient de noter que cette mise à jour ne concerne pas seulement les états de production mais aussi les états internes. Autrement dit, lorsqu'un état *s^{e,h,d}* devient actif, il convient de conclure que tous les états prédécesseurs dans l'arborescence, *ψ*(*s^{e,h,d}*), *ψ*²(*s^{e,h,d}*), *ψ*³(*s^{e,h,d}*), le sont également. La fonction *ψ* associe à tout état actif *s^{e,h,d}* d'un sous-modèle *HMM^{h,d}* son état prédécesseur actif dans l'arborescence, appartenant donc à un sous-modèle de niveau *d* - 1

Les probabilités conditionnelles d'émission p(**X***ₜ* |*zₜ*) pour les différents états de production sont calculées au moyen de la règle de Bayes, à partir des probabilités conditionnelles *a posteriori p*(*ψ^{j}*(*zₜ*)|**X***ₜ*) et des probabilités a *priori p*(*ψ^{j}(zₜ*)), soit :
[Math.5] $p \left({{X}_{_}}_{t}\left|{z}_{t}={s}^{e , h , d}\right.\right) = \frac{{\prod }_{j = 0}^{card \left({Z}_{t}\right)} p \left({ψ}^{j}\left({z}_{t}={s}^{e , h , d}\right)\left|{{X}_{_}}_{t}\right.\right) p \left({{X}_{_}}_{t}\right)}{{\prod }_{j = 0}^{card \left({Z}_{t}\right)} p \left({ψ}^{j}\left({z}_{t}={s}^{e , h , d}\right)\right)} ∝ \frac{{\prod }_{j = 0}^{card \left({Z}_{t}\right)} p \left({ψ}^{j}\left({z}_{t}={s}^{e , h , d}\right)\left|{{X}_{_}}_{t}\right.\right)}{{\prod }_{j = 0}^{card \left({Z}_{t}\right)} p \left({ψ}^{j}\left({z}_{t}={s}^{e , h , d}\right)\right)}$ Le module prédictif de la variable *zₜ*, prenant ses valeurs dans *S_{prod},* est entrainé de manière supervisée dans chaque phase de calibration *u* selon le même principe que le modèle prédictif de chaque expert.

On rappelle que le module prédictif de l'interface REW-MSLM de l'art antérieur fournit un modèle multilinéaire optimal *fₒₚₜ* parmi une pluralité *F* de modèles multilinéaires, respectivement définis par un ensemble de coefficients de prédiction ${\left\{{{B}_{_}}_{u}^{f}\right\}}_{f = 1}^{F}$, et un ensemble de vecteurs de biais de prédiction, ${\left\{{b}_{u}^{f}\right\}}_{f = 1}^{F}$, le rang *fₒₚₜ* conduisant à l'erreur de prédiction minimale étant sélectionné dans la phase de calibration.

Dans le cas présent, pour chaque sous-modèle *HMM^{h,d}*, l'algorithme REW-NPLS calcule une pluralité *F* de modèles multilinéaires définis par un ensemble de coefficients de prédiction ${\left\{{{B}_{_}}_{u}^{f , h , d}\right\}}_{f = 1}^{F}$, et un ensemble de vecteurs de biais de prédiction, ${\left\{{b}_{u}^{f , h , d}\right\}}_{f = 1}^{F}$*,* pour chacun des sous-modèles *HMM^{h,d}.* L'algorithme REW-NPLS sélectionne alors l'hyperparamètre *fₒₚₜ* et les modèles multilinéaires associés, soit $\left\{{{B}_{_}}_{u}^{fopt , h , d}, {b}_{u}^{fopt , h , d}\right\}$ conduisant à la plus faible erreur de prédiction, avec *d* = 1,.., *D* où est la profondeur de l'arborescence et *h* parcourant les valeurs d'indice des différents sous-modèles à chaque niveau *d*.

Les sous-modèles *HMM^{h,d}* ainsi mis à jour au terme de la phase de calibration *u*, définis par $\left\{{{B}_{_}}_{u}^{fopt , h , d}, {b}_{u}^{fopt , h , d}\right\}$, permettent de calculer les probabilités conditionnelles *p*(*ψ^{j}*(*zₜ* - *s^{e,h,d}*)|**X***ₜ*) intervenant dans l'expression (5) et d'en déduire les coefficients de pondération des experts associés aux différents états, à chaque instant *t*, *γ_{s,t}*. Pour des raisons de simplification de notation, on notera $\left\{{{B}_{_}}^{h , d}, {b}^{h , d}\right\} = \left\{{{B}_{_}}_{u}^{fopt , h , d}, {b}_{u}^{fopt , h , d}\right\}$, en sous-entendant que l'on prend implicitement en compte les modèles multilinéaires donnés par la dernière phase de calibration.

Il convient de rappeler que le coefficient de pondération *γ_{s,t}* exprime la probabilité que l'état de production *s* soit actif compte tenu de la séquence observée (historique du tenseur d'observation), **X**_{1:*t*}.

On suppose d'abord que les différents sous-modèles *HMM^{h,d}* sont indépendants. L'estimation de l'état actif de chaque sous-modèle *HMM^{h,d},* peut être représenté par un vecteur ŝ*^{h,d}* dont la taille est le nombre d'états du sous-modèle en question et les éléments sont représentatifs des probabilités d'activation des différents états *S^{e,h,d}* de ce sous-modèle, soit :
[Math. 6] ${\hat{s}}^{h , d} = {{B}_{_}}^{h , d} {{X}_{_}}_{t} + {b}^{h , d}$

Pour chaque état de production possible, *zₜ*, on calcule la probabilité d'activation des états dans chacun des sous-modèles des couches précédentes, *HMM^{h,d-j}, j* = 0, ..., *d* au moyen de la fonction *softmax*:
[Math. 7] $p \left({ψ}^{j}\left({z}_{t}\right)={s}^{e , h , d - j}\left|{{X}_{_}}_{t}\right.\right) = \frac{exp \left({\hat{s}}^{e , h , d - j}\right)}{{\sum }_{i = 1}^{{K}_{h , d}} exp \left({\hat{s}}^{i , h , d - j}\right)}$ où *K_{h,d-j}* est le nombre d'états possibles du sous-modèle *HMM^{h,d-j}.*

La probabilité conjointe de chaque état prédécesseur d'un état de production et de la séquence observée **X**_{1:*t*} est donnée par le processus markovien d'ordre 1 du sous-modèle *HMM^{h,d-j} à* savoir :
[Math. 8] $p \left({ψ}^{j}\left({z}_{t}\right)={s}^{e , h , d - j},{{X}_{_}}_{1 : t}\right) = p \left({ψ}^{j}\left({z}_{t}\right)={s}^{e , h , d - j}\left|{{X}_{_}}_{t}\right.\right) {\sum }_{i = 1}^{{K}_{k , d - j}} {a}_{e , i}^{h , d - j} p \left({ψ}^{j}\left({z}_{t - 1}\right)={s}^{i , h , d - j}\left|{{X}_{_}}_{1 : t - 1}\right.\right)$ où ${d}_{e , i}^{h , d - j}$ est la probabilité de transition entre les états *s ^{i,h,d-j}* et *s^{e,h,d-j}* du sous-modèle *HMM^{h,d-j}.*
La probabilité conditionnelle de chaque état prédécesseur d'un état de production connaissant la séquence observée X_{1:*t*} peut être alors être calculée à partir de :
[Math. 9] $p \left({ψ}^{j}\left({z}_{t}\right)={s}^{e , h , d - j}\left|{{X}_{_}}_{1 : t}\right.\right) = \frac{p \left({ψ}^{j}\left({z}_{t}\right)={s}^{e , h , d - j},{{X}_{_}}_{1 : t}\right)}{{\sum }_{i = 1}^{{K}_{k , d - i}} p \left({ψ}^{j}\left({z}_{t}\right)={s}^{i , h , d - j},{{X}_{_}}_{1 : t}\right)}$ Enfin, la probabilité *γ_{s,t}* = *p*(*zₜ* = *s^{e,h,d}* |**X**_{1:*t*}) que l'état de production *s^{e,h,d}* ∈ *S_{prod}* soit actif, compte tenu de la séquence observée **X**_{1*:t*}, peut être obtenue comme le produit des probabilités que les états internes et l'état de production soient actifs le long du chemin conduisant à *s^{e,h,d}* :
[Math. 10] ${γ}_{s , t} = p \left({z}_{t}=s\left|{{X}_{_}}_{1 : t}\right.\right) = {\sum }_{j = 0}^{d} p \left({ψ}^{j}\left({z}_{t}\right)={s}^{e , h , d - j}\left|{{X}_{_}}_{1 : t}\right.\right)$ où *s^{e,h,d - j}, j* = 0, ..., *d* décrivent les états le long du chemin conduisant à *s.* Ainsi, dans l'exemple illustré en Fig. 3B, on aura :
[Math. 11] $\begin{matrix}{γ}_{{s}^{1 , 1 , 1} , t} = p \left({ψ}^{0}\left({z}_{t}\right)={s}^{1 , 1 , 1}\left|{{X}_{_}}_{1 : t}\right.\right) ; \\ {γ}_{{s}^{1 , 1 , 2} , t} = p \left({z}_{t}={s}^{1 , 1 , 2}\left|{{X}_{_}}_{1 : t}\right.\right) = \\ p \left({ψ}^{0}\left({z}_{t}\right)={s}^{1 , 1 , 2}\left|{{X}_{_}}_{1 : t}\right.\right) . p \left({ψ}^{1}\left({z}_{t}\right)={s}^{2 , 1 , 1}\left|{{X}_{_}}_{1 : t}\right.\right) ; \\ {γ}_{{s}^{2 , 1 , 2} , t} = p \left({z}_{t}={s}^{2 , 1 , 2}\left|{{X}_{_}}_{1 : t}\right.\right) = \\ p \left({ψ}^{0}\left({z}_{t}\right)={s}^{2 , 1 , 2}\left|{{X}_{_}}_{1 : t}\right.\right) . p \left({ψ}^{1}\left({z}_{t}\right)={s}^{2 , 1 , 1}\left|{{X}_{_}}_{1 : t}\right.\right) ; \\ {γ}_{{s}^{1 , 2 , 2} , t} = p \left({z}_{t}={s}^{1 , 2 , 2}\left|{{X}_{_}}_{1 : t}\right.\right) = \\ p \left({ψ}^{0}\left({z}_{t}\right)={s}^{1 , 2 , 2}\left|{{X}_{_}}_{1 : t}\right.\right) . p \left({ψ}^{1}\left({z}_{t}\right)={s}^{3 , 1 , 1}\left|{{X}_{_}}_{1 : t}\right.\right) ; \\ {γ}_{{s}^{2 , 2 , 2} , t} = p \left({z}_{t}={s}^{2 , 2 , 2}\left|{{X}_{_}}_{1 : t}\right.\right) = \\ p \left({ψ}^{0}\left({z}_{t}\right)={s}^{2 , 2 , 2}\left|{{X}_{_}}_{1 : t}\right.\right) . p \left({ψ}^{1}\left({z}_{t}\right)={s}^{3 , 1 , 1}\left|{{X}_{_}}_{1 : t}\right.\right)\end{matrix}$ A partir des coefficients de pondération ainsi calculés, le tenseur de commande peut être estimé par une combinaison des prédictions des différents experts multilinéaires :
[Math. 12] ${{\hat{Y}}_{_}}_{t} = {\sum }_{s ∈ {S}_{prod}} {γ}_{s , t} \left({{B}_{_}}_{s}{{X}_{_}}_{t}+{{b}_{_}}_{s}\right)$ où Bₛ et **b**ₛ sont les paramètres de l'expert relativement à l'état de production S. Une seconde idée à la base de l'invention est d'utiliser dans le modèle H2M2, non plus des sous-modèles markoviens *HMM^{h,d}* mais des sous-modèles semi-markoviens ou HSMM *(Hidden Semi Markovian Model),* notés pour cette raison *HSMM^{h,d}*. Le modèle hiérarchique ainsi construit est alors noté H2SM2.

On appelle modèle semi-markovien un modèle markovien dans lequel les probabilités de transition entre états ne sont plus constantes mais dépendantes du temps. Plus précisément, la probabilité de transition, ${a}_{e , i}^{h , d}$, entre un état de départ *s^{e,h,d}* et un état d'arrivée *s^{i,h,d}* d'un sous-modèle *HSMM^{h,d}* est une fonction croissante du temps passé dans l'état de départ. On pourra choisir par exemple ${a}_{e , i}^{h , d} = {a}_{0} + \left(1-{a}_{0}\right) tanh \left({τ}_{e}^{h , d}\right)$ où 0 ≤ *a*₀ < 1 est la probabilité de transition à l'instant initial et ${τ}_{e}^{h , d}$ est le temps d'occupation de l'état de départ. Alternativement, on pourra choisir ${a}_{e , i}^{h , d} = {a}_{∞} \left(1-exp\left(-{τ}_{e}^{h , d}/T\right)\right)$ où *a*_{∞} est la probabilité de transition pour un temps infini. Alternativement encore, on pourra choisir ${a}_{e , i}^{h , d} = \frac{1}{2} \left(1-erf\left(\frac{{τ}_{e}^{h , d} - {μ}_{e}^{h , d}}{{σ}_{e}^{h , d} \sqrt{2}}\right)\right)$ où ${μ}_{e}^{h , d}$ est une durée moyenne de transition, ${σ}_{e}^{h , d}$ un écart-type et *erf*(.) est la fonction d'erreur de Gauss.
De manière générale, certains des sous-modèles du modèle H2SM2 pourront être semi-markoviens,*HSMM^{h,d}*, les autres étant simplement markoviens. De même dans un sous-modèle *HSMM^{h,d}* certaines transitions entre états pourront avoir une probabilité constante dans le temps, les autres dépendant du temps comme indiqué précédemment.
Si l'on note A*_{SM}*(*t*) la matrice de transitions entre états du modèle H2SM2 la probabilité qu'un état de production du modèle soit actif à l'instant *t* est donnée par :
[Math. 13] ${γ}_{t} = \left({A}_{SM}\left(t\right){γ}_{t - 1}\right) ∘ {γ}_{t}^{emission}$ où *γ_{s,t}* est un vecteur de taille *K* (nombre d'états du modèle) dont les éléments sont les probabilités *γ_{s,t}* respectives que les états soient actifs, ${γ}_{t}^{emission}$ est un vecteur de taille *K* donnant les probabilités d'émission des différents états et ∘ est le produit de Hadamard.

L'apprentissage du modèle H2SM2 diffère de celui du modèle H2M2 en ce que les paramètres d'évolution temporelle des transitions entre états (par exemple ${μ}_{e}^{h , d}$, ${σ}_{e}^{h , d}$, *a*_{∞}, *a*₀) doivent être estimés. Cet apprentissage peut être effectué dans une phase de calibration en mode supervisé ou semi-supervisé.

Selon la variante en mode d'apprentissage supervisé, l'utilisateur reçoit des instructions de cadence pour effectuer un ou des mouvement(s) de manière répétitive (ou imaginer ces mouvements lorsque l'utilisateur est handicapé moteur), le cas échéant à différentes cadences. La cadence est fournie à l'utilisateur sous la forme de stimuli extérieurs (visuels ou sonores par exemple).

Selon la variante en mode d'apprentissage semi-supervisé, la mise à jour des paramètres du modèle ne se limite pas à la phase de calibration mais se poursuit dans la phase opérationnelle. Dans ce cas, la cadence ou de manière plus générale les informations relatives aux transitions temporelles entre états sont fournies par la sortie du décodage.
La distinction entre états markoviens et états semi-markoviens peut résulter d'un choix a *priori.*
Alternativement, on peut supposer que tous les états sont semi-markoviens. La durée de chaque état semi-markovien est alors enregistrée pour chacune des instances d'apprentissage. Lorsqu'une durée est supérieure à un seuil prédéterminé, l'état est considéré comme markovien. A l'inverse, si elle est inférieure à ce seuil, les paramètres d'évolution temporelle des probabilités de transition sont estimés par moyennage (instances à cadence identique) ou par régression (instances d'apprentissage à différentes cadences).
La variante en mode d'apprentissage semi-supervisé opère de la même manière que dans la variante en mode supervisé, à ceci près que l'information de cadence est fournie par le décodeur lui-même. Dans un tel cas, l'utilisateur pourra être invité à effectuer différents mouvements de manière répétitive (marche par exemple) à différentes cadences, voire à penser ces mouvements lorsque l'utilisateur est handicapé moteur. Les paramètres d'évolution temporelle des probabilités de transition sont estimés et mis à jour à partir des durées respectives des états prédits par le modèle H2SM2.
Comme dans la première variante, une fois l'interface neuronale directe calibrée, les prédictions des différents experts sont combinées dans la phase opérationnelle pour estimer le tenseur de commande :
[Math. 14] ${\hat{{Y}_{_}}}_{t} = {\sum }_{s ∈ {S}_{prod}} {γ}_{{SM}_{s , t}} \left({{B}_{_}}_{s}{{X}_{_}}_{t}+{{b}_{_}}_{s}\right)$ où *γ*_{*SMs,t*,} s ∈ *S_{prod}* sont les coefficients de pondération des différents états de production du modèle H2SM2, c'est-à-dire les probabilités respectives d'occupation de ces états au cours du temps.

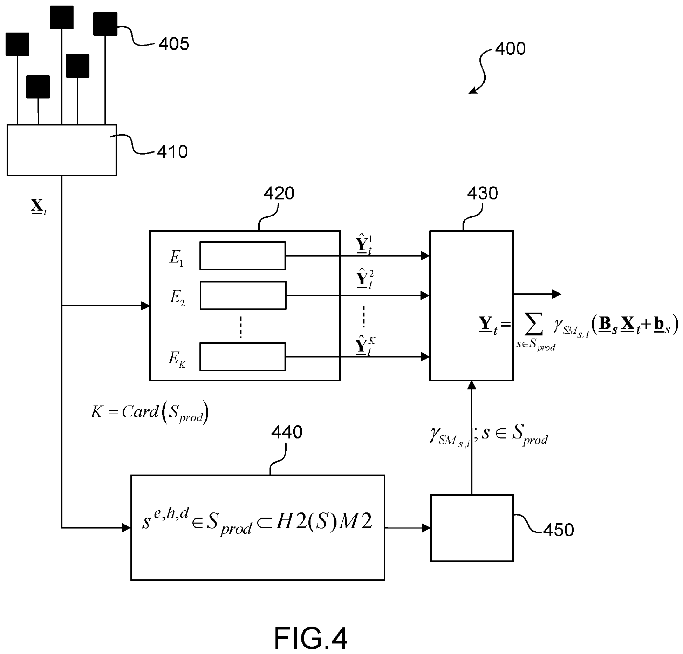

La Fig. 4 représente de manière schématique une interface neuronale directe utilisant un mélange d'experts contrôlé par un modèle hiérarchique (semi) markovien à états cachés selon un premier (resp. un second) mode de réalisation de l'invention.

L'interface BCI, 400, fait intervenir, d'une part un automate à états cachés, 440, obéissant à un modèle hiérarchique markovien (H2M2) ou semi-markovien (H2SM2) et, d'autre part, une pluralité d'experts, 420, chaque estimateur (ou expert) étant associé à un état de production du modèle H2(S)M2.

Les signaux électrophysiologiques issus des capteurs 405 sont échantillonnés et rassemblés par blocs de données, chaque bloc correspondant à une fenêtre d'observation glissante définie par un instant d'observation auquel démarre la fenêtre en question. Les signaux subissent un prétraitement dans le module de prétraitement 410, comprenant notamment une analyse temps-fréquence (décomposition en ondelettes sur les fenêtres d'observation, par exemple), pour fournir un tenseur d'observation, X*ₜ*, à chaque instant d'observation *t*.

Les estimations des différents experts sont pondérées à chaque instant d'observation par des coefficients de pondération, *γ_{s,t} γ_{SMs,t,} s* ∈ *S_{prod}* et combinées dans le module de combinaison 430 pour fournir une estimation du tenseur de commande selon l'expression (12) ou (14). Les coefficients de pondération donnent en chaque instant les probabilités d'occupation des différents états du modèle 440, sont estimés en chaque instant au moyen d'une régression REW-NPLS dans le module d'estimation 450. Les paramètres de cette régression sont déterminés dans une phase de calibration. Le cas échéant, les durées de vie des états semi-markoviens sont également estimées dans cette phase de calibration de manière supervisée ou semi-supervisée. En mode d'apprentissage semi-supervisé, l'estimation des durées de vie des états semi-markoviens peut s'étendre au-delà de la phase de calibration, durant la phase opérationnelle. Enfin, les paramètres (**B***ₛ*,**b***ₛ*) des estimateurs multilinéaires associés aux différents états de production sont déterminés sur des sous-séquences de calibration associées à ces états au moyen d'une régression REW-NPLS, de manière connue de l'homme du métier.

## Revendications

1. Interface neuronale directe configurée pour recevoir une pluralité de signaux électrophysiologiques émis par le cortex cérébral d'un usager acquis au moyen d'une pluralité de capteurs et pour fournir des signaux de commande décrivant une trajectoire à réaliser, lesdits signaux électrophysiologiques subissant un prétraitement pour obtenir un tenseur d'observation (**x**ₜ) en chaque instant d'observation, **caractérisé en ce que** l'évolution du tenseur d'observation est modélisée par un modèle Markovien caché, HMM, hiérarchisé, dit H2M2, comprenant une pluralité de sous-modèles HMM organisés selon une arborescence hiérarchique, ladite arborescence comportant à sa racine un modèle principal comprenant un état de repos , chaque état d'un sous-modèle HMM étant soit un état interne auquel est associé un sous-modèle HMM de plus bas niveau dans l'arborescence, soit un état de production, ladite interface utilisant un mélange d'experts, chaque expert (*Eₛ*) étant associé à un état de production et étant défini par un modèle prédictif multilinéaire, les prédictions de chaque expert étant combinées au moyen de coefficients de mélange (*γ_{s,t}*) pour fournir un tenseur de commande (**Y**ₜ) représentant lesdits signaux de commande.

2. Interface neuronale directe selon la revendication 1, **caractérisée en ce que** ladite arborescence comporte un état latéral droit et un état latéral gauche correspondant respectivement à la partie droite et à la partie gauche du corps de l'usager, une première branche de l'arborescence étant associée à l'état latéral droit et une seconde branche de l'arborescence étant associée à l'état latéral gauche.

3. Interface neuronale directe selon la revendication 1, **caractérisée en ce que** le tenseur de commande Y_{f} test estimé à partir du tenseur d'observation **X***ₜ* au moyen de ${{\hat{Y}}_{_}}_{t} = {\sum }_{s ∈ {S}_{prod}} {γ}_{s , t} \left({{B}_{_}}_{s}{{X}_{_}}_{t}+{{b}_{_}}_{s}\right)$ où **B**ₛ et **b**ₛ sont respectivement les tenseurs des coefficients de prédiction et des biais de prédiction du modèle prédictif multilinéaire définissant l'expert associé à l'état de production *s* et *γ_{s,t}* est un coefficient de mélange caractéristique de la probabilité d'occupation de l'état de production *s* 'à l'instant *t*.

4. Interface neuronale directe selon l'une des revendications 1 à 3, **caractérisée en ce que**, dans une phase de calibration, chacun des experts, *Eₛ*, est entrainé selon une méthode de régression Recursive Exponentially Weighted Markov Switching Multi-Linear Model, REW-NPLS, sur une sous-séquence du tenseur d'observation et du tenseur de commande de consigne, correspondant à l'état *s* ∈ *S_{prod}*.

5. Interface neuronale directe selon la revendication 4, **caractérisée en ce que**, dans ladite phase de calibration, les coefficients de mélange des différents experts sont estimés au moyen d'une méthode de régression REW-NPLS.

6. Interface neuronale directe selon la revendication 1, **caractérisée en ce qu'**au moins certains des sous-modèles HMM de l'arborescence sont semi-markoviens.

7. Interface neuronale directe selon la revendication 6, **caractérisée en ce que** le tenseur de commande **Y***_{f}* test estimé à partir du tenseur d'observation X*_{f}* au moyen de ${\hat{{Y}_{_}}}_{t} = {\sum }_{s ∈ {S}_{prod}} {γ}_{{SM}_{s , t}} \left({{B}_{_}}_{s}{{X}_{_}}_{t}+{{b}_{_}}_{s}\right)$ où Bₛ et **b**ₛ sont respectivement les tenseurs des coefficients de prédiction et des biais de prédiction du modèle prédictif multilinéaire définissant l'expert associé à l'état de production *s* et *γ_{SMs,t}* est un coefficient de mélange caractéristique de la probabilité d'occupation de l'état de production Sà l'instant *t*, la probabilité qu'un état de production du modèle soit actif à l'instant *t* étant donnée par ${γ}_{t} = \left({A}_{SM}\left(t\right){γ}_{t - 1}\right) ∘ {γ}_{t}^{emission}$ où *γ_{s,t}* est un vecteur dont les éléments sont les probabilités *γ_{s,t}* respectives que les états de production soient actifs, ${γ}_{t}^{emission}$ est un vecteur donnant les probabilités d'émission des différents états de production et ∘ est le produit de Hadamard.

8. Interface neuronale directe selon la revendication 7, **caractérisée en ce que** la probabilité de transition entre un état de départ et un état d'arrivée d'un sous-modèle HMM semi-markovien est d'autant plus élevée que la durée d'occupation de l'état de départ est plus grande.

9. Interface neuronale directe selon la revendication 8, **caractérisée en ce que** les probabilités de transition entre états d'un sous-modèle HMM semi-markovien évoluent dans le temps selon une loi paramétrée par une pluralité de paramètres d'évolution temporelle, lesdits paramètres d'évolution temporelle étant estimés dans une phase de calibration supervisée pendant laquelle l'usager reçoit des instructions de cadence prédéterminée pour effectuer un ou des mouvement(s) de manière répétitive, la durée d'occupation de chaque état du sous-modèle HMM semi-markovien étant enregistrée au cours du temps.

10. Interface neuronale directe selon la revendication 8, **caractérisée en ce que** les probabilités de transition entre états d'un sous-modèle HMM semi-markovien évoluent dans le temps selon une loi paramétrée par une pluralité de paramètres d'évolution temporelle, lesdits paramètres d'évolution temporelle étant estimés, dans une phase de calibration semi-supervisée et/ou une phase opérationnelle , à partir du tenseur d'observation, la durée d'occupation de chaque état du sous-modèle HMM semi-markovien étant enregistrée au cours du temps.

11. Interface neuronale directe selon l'une des revendications précédentes, **caractérisée en ce que** les signaux électrophysiologiques sont des signaux ECoG et que les signaux de commande sont destinés à commander les actuateurs d'un exosquelette.

## Patentansprüche

1. **Direkte** neuronale Schnittstelle, die dazu ausgelegt ist, eine Mehrzahl von elektrophysiologischen Signalen zu empfangen, die von der Großhirnrinde eines Benutzers ausgesendet werden und mittels einer Mehrzahl von Sensoren erfasst werden, uund Steuersignale bereitzustellen, die eine zu realisierende Trajektorie beschreiben, wobei die elektrophysiologischen Signale einer Vorverarbeitung unterzogen werden, um einen Beobachtungssensor (Xₜ) z jedem Beobachtungszeitpunkt zu erhalten, **dadurch gekennzeichnet, dass** der Verlauf des Beobachtungssensors durch ein als H2M2 bezeichnetes hierarchisiertes Hidden-Markov-Modell HMM modelliert wird, das eine Mehrzahl von HMM-Untermodellen umfasst, die gemäß einer hierarchischen Baumstruktur organisiert sind, wobei die Baumstruktur an ihrer Wurzel ein Hauptmodell aufweist, das einen Ruhezustand umfasst, wobei jeder Zustand eines HMM-Untermodells entweder ein interner Zustand, dem ein HMM-Untermodell einer niedrigeren Ebene in der Baumstruktur zugeordnet ist, oder ein Produktionszustand ist, wobei die Schnittstelle eine Expertenmischung verwendet, wobei jeder Experte (*Eₛ*) einem Produktionszustand zugeordnet ist und durch ein multilineares prädiktives Modell definiert wird, wobei die Prädiktionen jedes Experten mittels Mischungskoeffizienten (γ_{s,t}) kombiniert werden, um einen Steuertensor (Yₜ) bereitzustellen, der die Steuersignale repräsentiert.

2. Direkte neuronale Schnittstelle nach Anspruch 1, **dadurch gekennzeichnet, dass** die Baumstruktur einen rechten seitlichen Zustand und einen linken seitlichen Zustand aufweist, die dem rechten bzw. dem linken Körperteil des Benutzers entsprechen, wobei ein erster Zweig der Baumstruktur dem rechten seitlichen Zustand zugeordnet ist und wobei ein zweiter Zweig der Baumstruktur dem linken seitlichen Zustand zugeordnet ist.

3. Direkte neuronale Schnittstelle nach Anspruch 1, **dadurch gekennzeichnet, dass** der Steuertensor Y*_{f}* ausgehend von dem Beobachtungssensor X*_{f}* mittels ${\hat{{Y}_{_}}}_{t} = {\sum }_{s ∈ {S}_{prod}} {γ}_{s , t} \left({{B}_{_}}_{s}{{X}_{_}}_{t}+{{b}_{_}}_{s}\right)$ geschätzt wird, worin Bₛ und bₛ die Tensoren der Prädiktionskoeffizienten bzw. der Prädiktionsbias des multilinearen prädiktiven Modells sind, das den Experten definiert, der dem Produktionszustand s zugeordnet ist, und γ_{s,t} ein Mischungskoeffizient ist, der für die Wahrscheinlichkeit des Einnehmens des Produktionszustands s zu dem Zeitpunkt t charakteristisch ist.

4. Direkte neuronale Schnittstelle nach einem der Ansprüche 1 bis 3, **dadurch gekennzeichnet, dass** in einer Kalibrierungsphase jeder der Experten, Eₛ, nach einer **Regressionsmethode** Recursive Exponentially Weighted Markov Switching Multi-Linear Model, REW-NPLS, über eine Untersequenz des Beobachtungstensors und des Soll-Steuertensors, entsprechend dem Zustand *s* ∈ *S_{prod}*, trainiert wird.

5. Direkte neuronale Schnittstelle nach Anspruch 4, **dadurch gekennzeichnet, dass** in der Kalibrierungsphase die Mischungskoeffizienten der verschiedene Experten mittels einer Regressionsmethode REW-NPLS geschätzt werden.

6. Direkte neuronale Schnittstelle nach Anspruch 1, **dadurch gekennzeichnet, dass** mindestens einige der HMM-Untermodelle der Baumstruktur Semi-Markov-Modelle sind.

7. Direkte neuronale Schnittstelle nach Anspruch 6, **dadurch gekennzeichnet, dass** der Steuertensor Y*_{f}* ausgehend von dem Beobachtungssensor X*_{f}* mittels ${\hat{{Y}_{_}}}_{t} = {\sum }_{s ∈ {S}_{prod}} {γ}_{{SM}_{s , t}} \left({{B}_{_}}_{s}{{X}_{_}}_{t}+{{b}_{_}}_{s}\right)$ geschätzt wird, worin Bₛ und bₛ die Tensoren der Prädiktionskoeffizienten bzw. der Prädiktionsbias des multilinearen prädiktiven Modells sind, das den Experten definiert, der dem Produktionszustand s zugeordnet ist, und *γ_{SMs,t}* ein Mischungskoeffizient ist, der charakteristisch für die Wahrscheinlichkeit des Einnehmens des Produktionszustands s zu dem Zeitpunkt t ist, wobei die Wahrscheinlichkeit, dass ein Produktionszustand des Modells zu dem Zeitpunkt t aktiv ist, durch ${γ}_{t} = \left({A}_{SM}\left(t\right){γ}_{t - 1}\right) ∘ {γ}_{t}^{emission}$ gegeben wird, worin γ*_{s,t}* ein Vektor ist, dessen Elemente die jeweiligen Wahrscheinlichkeiten *γ_{s,t}* sind, dass die Produktionszustände aktiv sind, ${γ}_{t}^{emission}$ ein Vektor ist, der die Wahrscheinlichkeiten des Aussendens der verschiedenen Produktionszustände angibt, und o das Hadamard-Produkt ist.

8. Direkte neuronale Schnittstelle nach Anspruch 7, **dadurch gekennzeichnet, dass** die Wahrscheinlichkeit des Übergangs zwischen einem Ausgangszustand und einem Zielzustand eines Semi-Markov-Untermodells HMM umso höher ist, je größer die Dauer des Einnehmens des Ausgangszustands ist.

9. Direkte neuronale Schnittstelle nach Anspruch 8, **dadurch gekennzeichnet, dass** sich die Wahrscheinlichkeiten des Übergangs zwischen Zuständen eines Semi-Markov-Untermodells HMM im Laufe der Zeit nach einem Gesetz entwickeln, das durch eine Mehrzahl von Parametern für den zeitlichen Verlauf parametriert wird, wobei die Parameter für den zeitlichen Verlauf in einer überwachten Kalibrierungsphase geschätzt werden, während der der Benutzer Anweisungen mit vorbestimmter Taktung empfängt, um eine oder mehrere Bewegung(en) wiederholt auszuführen, wobei die Dauer des Einnehmens jedes Zustands des Semi-Markov-Untermodells HMM während der Zeit aufgezeichnet wird.

10. Direkte neuronale Schnittstelle nach Anspruch 8, **dadurch gekennzeichnet, dass** sich die Wahrscheinlichkeiten des Übergangs zwischen Zuständen eines Semi-Markov-Untermodells HMM im Laufe der Zeit nach einem Gesetz entwickeln, das durch eine Mehrzahl von Parametern für den zeitlichen Verlauf parametriert wird, wobei die Parameter für den zeitlichen Verlauf in einer halbüberwachten Kalibrierungsphase und/oder einer Betriebsphase ausgehend von dem Beobachtungssensor geschätzt werden, wobei die Dauer des Einnehmens jedes Zustands des Semi-Markov-Untermodells HMM während der Zeit aufgezeichnet wird.

11. Direkte neuronale Schnittstelle nach einem der vorhergehenden Ansprüche, **dadurch gekennzeichnet, dass** die elektrophysiologischen Signale ECoG-Signale sind und dass Steuersignale dazu bestimmt sind, die Aktoren eines Exoskeletts zu steuern.

## Claims

1. A direct neural interface designed to receive a plurality of electrophysiological signals emitted by a user's cerebral cortex and acquired by means of a plurality of sensors, and to provide control signals describing a trajectory to be followed, said electrophysiological signals undergoing pre-processing to obtain an observation tensor (X*ₜ*) at each observation time, **characterised in that** the evolution of the observation tensor is modelled by a hierarchical HMM model, known as H2M2, comprising a plurality of HMM sub-models organised according to a hierarchical tree structure, said tree structure having at its root a main model comprising a resting state, each state of an HMM sub-model being either an internal state to which a lower-level HMM sub-model in the tree is associated, or a production state, said interface using a mixture of experts, each expert (*Eₛ*) being associated with a production state and being defined by a multilinear predictive model, the predictions of each expert being combined by means of mixing coefficients (*γ_{s,t},γ_{SMs,t ;} s* ∈ *S_{prod})* to provide a control tensor (**Ŷ***ₜ*) representing said control signals.

2. Direct neural interface as claimed in claim 1, **characterised in that** said tree structure comprises a right lateral state and a left lateral state corresponding respectively to the right and left sides of the user's body, a first branch of the tree structure being associated with the right lateral state and a second branch of the tree structure being associated with the left lateral state.

3. Direct neural interface as claimed in claim 1, **characterised in that** the control tensor **Y***ₜ* is estimated from the observation tensor **X***ₜ* by means of ${{\hat{Y}}_{_}}_{t} = {\sum }_{s ∈ {S}_{prod}} {γ}_{s , t} \left({{B}_{_}}_{s}{{X}_{_}}_{t}+{{b}_{_}}_{s}\right)$ where Bₛ and **b**ₛ are, respectively, the prediction coefficient and prediction bias tensors of the multilinear predictive model defining the expert associated with the production state *s* and *γ_{s,t}* is a mixing coefficient characteristic of the the production state *s* occupancy probability at time *t.*

4. Direct neural interface according to any one of claims 1 to 3, **characterised in that**, during a calibration phase, each of the experts, *Eₛ*, is trained using an REW-NPLS regression method on a subsequence of the observation tensor and the control tensor corresponding to the state *s* ∈ *S_{prod}*.

5. Direct neural interface according to claim 4, **characterised in that**, during the said calibration phase, the mixing coefficients of the various experts are estimated using an REW-NPLS regression method.

6. Direct neural interface according to claim 1, **characterised in that** at least some of the HMM sub-models in the tree structure are semi-Markovian.

7. Direct neural interface according to claim 6, **characterised in that** the control tensor Y*ₜ* is estimated from the observation tensor **X***ₜ* using ${\hat{{Y}_{_}}}_{t} = {\sum }_{s ∈ {S}_{prod}} {γ}_{{SM}_{s , t}} \left({{B}_{_}}_{s}{{X}_{_}}_{t}+{{b}_{_}}_{s}\right)$ where **B**ₛ and **b**ₛ are, respectively, the prediction coefficient tensor and the prediction bias tensor of the multilinear predictive model defining the expert associated with the production state *s* and *γ_{SMs,t}* is a mixing coefficient characterising the probability of the production state *s* being active at time *t*, the probability that a production state of the model is active at time *t* being given by ${γ}_{t} = \left({A}_{SM}\left(t\right){γ}_{t - 1}\right) ∘ {γ}_{t}^{emission}$, where γ*_{s,t}* Is a vector whose elements are the respective probabilities *γ_{s,t}* that the production states are active, ${γ}_{t}^{emission}$ is a vector giving the emission probabilities of the various production states, and ∘ is the Hadamard product.

8. Direct neural interface according to claim 7, **characterised in that** the probability of transition between a start state and an end state of a semi-Markovian HMM sub-model is higher the longer the start state is occupied.

9. Direct neural interface according to claim 8, **characterised in that** the transition probabilities between states of a semi-Markovian HMM sub-model evolve over time according to a distribution parameterised by a plurality of temporal evolution parameters, said temporal1-evolution parameters being estimated during a supervised calibration phase during which the user receives instructions at a predetermined rate to perform one or more movements repeatedly, the duration of occupancy of each state of the semi-Markovian HMM sub-model being recorded over time.

10. Direct neural interface according to claim 8, **characterised in that** the transition probabilities between states of a semi-Markovian HMM sub-model evolve over time according to a distribution parameterised by a plurality of temporal evolution parameters, said time-evolution parameters being estimated, during a semi-supervised calibration phase and/or an operational phase, from the observation tensor, the duration of occupancy of each state of the semi-Markovian HMM sub-model being recorded over time.

11. Direct neural interface according to one of the preceding claims, **characterised in that** the electrophysiological signals are ECoG signals and the control signals are intended to control the actuators of an exoskeleton.
